(19) 

Europäisches
Patentamt

European
Patent Office

Office européen
des brevets

(11) **EP 2 435 030 B1**

(12) **FASCICULE DE BREVET EUROPEEN**

(45) Date de publication et mention
de la délivrance du brevet:
**26.11.2014 Bulletin 2014/48**

(21) Numéro de dépôt: **10728844.1**

(22) Date de dépôt: **28.05.2010**

(51) Int Cl.:
***A61K 9/50*** *(2006.01)*     ***A61K 9/00*** *(2006.01)*

(86) Numéro de dépôt international:
**PCT/FR2010/051038**

(87) Numéro de publication internationale:
**WO 2010/136739 (02.12.2010 Gazette 2010/48)**

(54) **COMPOSITIONS PHARMACEUTIQUES FLOTTANTES À LIBÉRATION CONTRÔLÉE**

SCHWEBENDE PHARMAZEUTISCHE ZUSAMMENSETZUNGEN MIT KONTROLLIERTER
FREISETZUNG

CONTROLLED-RELEASE FLOATING PHARMACEUTICAL COMPOSITIONS

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **29.05.2009 FR 0953601**

(43) Date de publication de la demande:
**04.04.2012 Bulletin 2012/14**

(73) Titulaire: **Flamel Technologies
69200 Vénissieux (FR)**

(72) Inventeurs:
• **CASTAN, Catherine
F-69530 Orlienas (FR)**
• **CAISSE, Philippe
F-69720 St Bonnet de Mure (FR)**

(74) Mandataire: **Cabinet Plasseraud
235 Cours Lafayette
69006 Lyon (FR)**

(56) Documents cités:
**EP-B1- 1 524 968    WO-A1-01/58424
WO-A1-99/01112    WO-A2-2006/131566**

• **TAKENAKA H ET AL: "Polymorphism of spraydried microencapsulated sulfamethoxazole with
cellulose acetate phthalate and colloidal silica,
montmorillonite, or talc", JOURNAL OF
PHARMACEUTICAL SCIENCES, vol. 70, no. 11,
novembre 1981 (1981-11), pages 1256-1260,
XP002155100,**

**Description**

**DOMAINE DE L'INVENTION**

La présente invention concerne des compositions pharmaceutiques ou systèmes multiparticulaires flottants et leur procédé de préparation. L'invention concerne également l'application de ces systèmes flottants pour la libération contrôlée de substances actives pharmaceutiques, notamment celles ayant une fenêtre d'absorption située dans la partie haute de l'estomac.

**ETAT DE LA TECHNIQUE**

De nombreux travaux ont été réalisés pour améliorer l'efficacité des compositions pharmaceutiques, notamment par le contrôle du profil de libération des principes actifs pharmaceutiques qu'elles contiennent. Cependant, même si la durée de libération peut être fortement allongée ou décalée, la durée d'action de ces préparations pharmaceutiques, et donc leur efficacité, est fortement dépendante du temps de séjour dans l'estomac, notamment lorsque le principe actif est absorbé seulement dans les parties hautes du tractus gastro-intestinal. La relative courte durée, la grande variabilité et la non prédictibilité du temps de séjour dans l'estomac sont des obstacles majeurs dans l'optimisation de l'efficacité de ces formes.

Diverses solutions ont été proposées pour augmenter le temps de séjour dans l'estomac. On peut citer par exemple les systèmes gastrorétentifs qui, en présence des fluides gastriques, se dilatent suffisamment ou se déplient pour atteindre une taille supérieure à celle de l'ouverture du pylore, les systèmes muco-adhésifs qui adhèrent à la paroi de l'estomac, les systèmes dont la densité élevée les fait se maintenir dans la partie basse du sac gastrique. Enfin, on peut citer les systèmes flottants dont la faible densité les maintient dans la partie supérieure du contenu stomacal.

La demande de brevet WO 01/58424 (West Pharma) décrit un ensemble de particules flottantes comprenant un coeur contenant un actif, puis un pelliculage de polymère entérique puis une couche de poudre de chitosan déposée par mélange, et une couche hydrophobe composée de stéarate de magnésium. Les performances de flottaison sont évaluées avec un test dans 500 mL d'acide chlorhydrique 0,1 N pendant 30 min, sans agitation et en l'absence de tensio-actif.

D'autres travaux ont porté sur des systèmes flottants multiparticulaires. Rouge N. et al. dans Pharm. Acta Helv. 73(2) 81-87 (1998) ont décrit des minicomprimés à base de polymère hydrophile gonflant (HPMC) de 3 mm de diamètre incorporés dans une gélule. D'autres encore ont préparé des microparticules en utilisant divers supports poreux de faible densité tels que des microsphères de polycarbonate (Joseph NJ et al. (Journal of Controlled Released (2002) 79, 71-79), des microsphères de silicate de calcium (Jain Sunil K. et al. (Drug Development and Industrial Pharmacy (2007) 33, p.381-391), des particules de polypropylène (Streubel A. et al. (International Journal of Phannaceutics 198 (2002), 279-292), des billes d'alginate de calcium (Choi BY et al. (International Journal of Pharmaceutics 239 (2002) 81-91) ou des billes de gélatine et caséine (Bulgarelli E. et al., International Journal of Pharmaceutics 198 (2000),157-165).

Dans le brevet FR2263744 (Eisai Co) ce sont des minicomprimés de polystyrène (6mm x 9mm) ou des grains de riz soufflé qui sont utilisés comme supports flottants, mais le volume relativement important de ces produits ne permet pas d'utiliser un grand nombre de supports par administration. De plus ces matériaux ne sont pas homologués pour une utilisation pharmaceutique.

Les déposants du brevet US 5,626,876 (Muller - Lohmann Therapie Système - 1997) utilisent des éléments poreux pour obtenir des formes pharmaceutiques flottantes. Notamment, un des modes de réalisation décrit une pluralité de sous-systèmes flottants composés d'une particule de mousse, recouverte d'une couche contenant un actif pharmaceutique, et éventuellement une seconde couche de pelliculage conférant un contrôle de la libération. Ces particules de mousse sont basées sur les polymères expansés de type polystyrène, polyamide ou polypropylène, commercialisés sous la marque Accurel (Annak Company, Chicago maintenant Akzo Chemicals). Ces polymères ne sont pas autorisés pour une administration orale et sont considérés par le fabricant comme des microéponges capables d'absorber plusieurs fois leur poids d'un liquide qui peut être l'eau. Ils sont donc non flottants. En effet, en présence d'un milieu liquide, les pores, qui sont des pores ouverts, se remplissent, la densité du matériau est augmentée et les particules coulent. Un principe actif ne peut donc être déposé que par voie sèche, sauf à déposer préalablement à leur surface une couche imperméabilisante comme mentionné en colonne 2 lignes 55-57. De plus aucune indication quantitative de la capacité de flottaison des divers produits finis décrits dans le brevet n'est donnée.

Des microsphères flottantes composées d'un coeur contenant un principe actif pharmaceutique et un polymère matriciel, entouré d'une couche intermédiaire contenant au moins un excipient non soluble (hydrophobe ou gras) et poreux, préférentiellement du béhénate de glycérol ou du phosphate de calcium dibasique, et un film composé d'un polymère permettant la libération contrôlée du principe actif, sont décrites dans la demande de brevet WO 2006/063858 (US 2008/0317841 Grenier et al.). De nouveau aucune indication quantitative de la durée de flottaison de ces microparticules n'est donnée dans la demande de brevet.

Une autre solution pour réaliser des systèmes flottants consiste à générer du gaz in situ pour diminuer la densité des comprimés ou granules (US 4,844,905, US 4,101,650, WO 2007/010400, US 6,261,601). Généralement ces systèmes incluent un sel alcalin de carbonate qui au contact des sucs gastriques acides, et parfois grâce à la présence d'un acide dans la formulation, génère in situ du gaz carbonique. Ce dernier est piégé dans la matrice hydrophile gélifiée qui compose la forme pharmaceutique, basée sur des polymères tels que l'hydroxypropylméthyl cellulose, ou des polysaccharides tels que les gommes xanthane, caroube, guar, ou les dérivés du chitosan. La demande de brevet WO 02/105415 décrit des systèmes monolithiques ou multiparticulaires flottants effervescents uniquement basés sur de la poudre de graines de cresson *Lepidium sativum.* De tels systèmes effervescents ne sont pas souhaités, car il est toujours très difficile de régler précisément le taux d'effervescence, surtout dans des microparticules, et donc d'avoir une flottabilité constante et reproductible entre les différentes particules.

Il existe donc un réel besoin en des systèmes flottants multiparticulaires, faciles à mettre en oeuvre et économiques qui permettent, de manière simple, précise et reproductible d'adapter le profil de libération au temps de séjour prolongé dans l'estomac.

## BREVE DESCRIPTION DE L'INVENTION

Il est du mérite de la demanderesse d'avoir répondu à ce besoin par des compositions pharmaceutiques comprenant une pluralité de microparticules comportant chacune un coeur flottant sur lequel est déposée une couche de principe actif recouverte d'une couche d'enrobage à libération contrôlée.

La composition pharmaceutique de l'invention est multiparticulaire, c'est-à-dire qu'elle est constituée d'un grand nombre de microparticules (par exemple plusieurs centaines voire plusieurs milliers). Cette multiplicité assure statistiquement une bonne reproductibilité de la cinétique de transit du principe actif dans tout le tractus gastrointestinal, il en résulte un meilleur contrôle de la biodisponibilité et donc une meilleure efficacité. Compte tenu de cette pluralité de microparticules, il est aisé de combiner des microparticules contenant différents principes actifs, chacun pouvant être libéré avec une cinétique différente et indépendante des autres.

La composition pharmaceutique selon invention comprenne une pluralité de microparticules enrobées a libération contrôlée comportant chacune un coeur flottant, à la surface duquel est déposée une couche contenant au moins un principe actif, ladite couche étant recouverte d'un enrobage a libération contrôlée, caractérisée par le fait que ledit coeur flottant comprend au moins 50% en poids d'acétate phtalate de cellulose et présente une masse volumique apparente après tassement comme décrit dans la pharmacopée européenne, édition 6.4, au chapitre 02.09.34, inférieure ou égale à 0,6 g/mL et lesdites microparticules enrobées présentent une masse volumique apparente après tassement comme décrit dans la pharmacopée européenne, édition 6.4, au chapitre 02.09.34, inférieure ou égale à 0,7 g/mL.

La composition selon l'invention, une fois administrée par voie orale, libère des microparticules (ou granules) enrobés qui se maintiennent dans la partie haute du contenu gastrique de telle sorte que leur temps de séjour dans l'estomac est prolongé. L'enrobage des granules est adapté à une libération contrôlée du principe actif qui tient compte de ce temps de séjour dans l'estomac.

La composition selon l'invention possède des avantages par rapport à des systèmes basés sur des matrices gonflantes et gélifiantes, voire effervescentes dans l'estomac: d'une part, les dimensions des microparticules enrobées restent pratiquement constantes ce qui permet un contrôle et une reproductibilité de la libération, d'autre part la flottaison est immédiate et ne nécessite pas un temps d'hydratation, de gonflement ou de réaction effervescente pendant lequel la forme ne flotterait pas et risquerait donc d'être expulsée par le pylore.

## DESCRIPTION DETAILLEE DE L'INVENTION

Ainsi l'invention a pour objet une composition pharmaceutique comprenant une pluralité de microparticules enrobées à libération contrôlée comportant chacune un coeur flottant, à la surface duquel est déposée une couche contenant au moins un principe actif, ladite couche étant recouverte d'un enrobage à libération contrôlée, ledit coeur flottant présentant une masse volumique apparente inférieure ou égale à 0,6 g/mL, de préférence inférieure ou égale à 0,5 g/mL et plus préférentiellement encore inférieure ou égale à 0,4 g/mL, et lesdites microparticules enrobées présentant une masse volumique apparente inférieure ou égale à 0,7 g/mL, de préférence inférieure ou égale à 0,6 g/mL et plus préférentiellement encore inférieure ou égale à 0,5 g/mL.

L'invention a plus particulièrement pour objet une composition pharmaceutique comprenant une pluralité de microparticules enrobées à libération contrôlée comportant chacune un coeur flottant, à la surface duquel est déposée une couche contenant au moins un principe actif, ladite couche étant recouverte d'un enrobage à libération contrôlée, caractérisée par le fait que ledit coeur flottant est composé d'acétate phtalate de cellulose et présente une masse volumique apparente inférieure ou égale à 0,6 g/mL et lesdites microparticules enrobées présentent une masse volumique apparente inférieure ou égale à 0,7 g/mL.

L'invention a également pour objet une composition pharmaceutique comprenant une pluralité de microparticules enrobées à libération contrôlée comportant chacune un coeur flottant, à la surface duquel est déposée une couche contenant au moins un principe actif, ladite couche étant recouverte d'un enrobage à libération contrôlée, caractérisée par le fait que ledit coeur flottant est composé d'acétate phtalate de cellulose.

Les coeurs flottants selon l'invention présentent avantageusement une porosité fermée supérieure ou égale à 0,2, de préférence supérieure ou égale à 0,4, de préférence supérieure ou égale à 0,6.

Les coeurs flottants présentent avantageusement une flottabilité F telle que mesurée selon le test de flottaison décrit ci-après dans le chapitre TESTS, supérieure ou égale à 50% après 1 heure, de préférence supérieure ou égale à 50% après 4 heures, plus préférentiellement supérieure ou égale à 50% après 6 heures et encore plus préférentiellement supérieure ou égale à 50% après 8 heures.

Les différentes masses volumiques s'entendent selon la définition donnée dans la pharmacopée européenne, édition 6.4, monographie 02.02.42. Plus précisément on entend par :

Masse volumique vraie ( $\rho_v$ ): cette masse volumique ne prend en compte que le volume occupé par le solide à l'exclusion des vides intra ou interparticulaires. Cette masse volumique est mesurée à l'aide d'un pycnomètre à hélium, le gaz hélium pénétrant les pores fermés.

Masse volumique particulaire ( $\rho_p$ ): cette masse volumique prend en compte le volume occupé par le solide et le volume des pores fermés des particules. On y accède à l'aide d'un pycnomètre à liquide selon le test de mesure de la masse volumique particulaire décrit ci-dessous en relation avec le chapitre TESTS.

Masse volumique apparente ($\rho_a$): la masse volumique apparente obtenue après tassement du lit de poudre selon le test de mesure de la masse volumique apparente après tassement décrit ci-dessous en relation avec le chapitre TESTS. Le volume considéré comprend le volume de la particule et le volume intraparticulaire du lit de poudre tassée.

Au sens de l'invention et dans tout le présent exposé, le terme « principe actif » couvre non seulement le principe actif en tant que tel mais aussi ses sels, ses énantiomères, ses isomères, ses solvates ou ses formes polymorphes.

De façon indifférente, on utilise ou bien le terme "enrobage" ou bien le terme "pelliculage" pour désigner la couche contrôlant la libération du principe actif.

L'enrobage est dit « à libération contrôlée » s'il permet de modifier, retarder ou prolonger la libération du principe actif. De tels enrobages ont largement été décrits dans la littérature et ceux qui sont tout particulièrement adaptés à l'invention sont ceux décrits notamment dans les demandes de brevet EP 709 087, WO 03/030878, EP 1 524 968, EP 1 524 969 et PCT/FR2009/050719.

Selon l'invention, on entend par « coeur flottant composé d'acétate phtalate de cellulose » un coeur flottant qui comprend au moins 50 % en poids d'acétate phtalate de cellulose, de préférence au moins 60 % en poids, de préférence au moins 70 % en poids, de préférence au moins 80 % en poids, de préférence au moins 90 % en poids, de préférence au moins 95% en poids, de préférence au moins 98 % en poids d'acétate phtalate de cellulose. Dans un mode de réalisation particulièrement avantageux, le coeur flottant est essentiellement constitué d'acétate phtalate de cellulose.

**Coeurs flottants:**

Lesdits coeurs flottants sont compatibles avec une administration orale de la forme pharmaceutique.

Les coeurs flottants utilisés présentent une masse volumique apparente inférieure ou égale à 0,6 g/mL, de préférence inférieure ou égale à 0,5 g/mL, et plus préférentiellement encore inférieure ou égale 0,4 g/mL.

Selon un mode de réalisation particulier, les coeurs flottants selon l'invention présentent une porosité fermée telle que la masse volumique particulaire est toujours intérieure à la masse volumique vraie. La porosité fermée est calculée comme suit :

$$\text{Porosité fermée} = 1 - \frac{\rho_p}{\rho_v}$$

Selon un autre mode de réalisation particulier, les coeurs flottants selon l'invention présentent une porosité fermée supérieure ou égale à 0,2, de préférence supérieure ou égale à 0,4, de préférence supérieure ou égale à 0,6.

Selon un autre mode de réalisation de l'invention, les coeurs flottants sont poreux.

Selon un autre mode de réalisation de l'invention, les coeurs flottants présentent une flottabilité F telle que mesurée selon le test de flottaison décrit ci-après dans le chapitre TESTS, supérieure ou égale à 50% après 1 heure, de préférence supérieure ou égale à 50% après 4 heures, plus préférentiellement supérieure ou égale à 50% après 6 heures et encore plus préférentiellement supérieure ou égale à 50% après 8 heures.

Les coeurs flottants mis en oeuvre dans les microparticules de la composition de l'invention, ne présentent sensiblement pas de porosité ouverte, ils permettent donc le dépôt de principe actif directement par voie humide sans nécessiter de couche intermédiaire.

Selon un mode de réalisation préféré, le diamètre moyen équivalent en volume des coeurs flottants est compris entre 50 μm et 4000 μm. Une opération de tamisage selon les techniques conventionnelles connues par l'homme de l'art peut permettre de sélectionner des coupes granulométriques définies. Plus particulièrement, lesdits coeurs flottants peuvent avoir un diamètre moyen équivalent en volume compris dans l'une des plages suivantes : 50 à 500 μm, 500 à 1000 μm ou 1000 à 4000 μm.

Dans un mode de réalisation particulier, les coeurs flottants présentent une résistance mécanique suffisante pour résister notamment aux efforts de cisaillement, de friction et de chocs auxquels ils seront soumis au cours du procédé de préparation des compositions pharmaceutiques selon l'invention. En particulier, selon un mode de réalisation avantageux la couche de principe actif sera déposée par pulvérisation, en utilisant des techniques conventionnelles dans un appareil à lit d'air fluidisé équipé d'un tube Wurster ou d'un rotogranulator (par exemple un GPCG1 de Glatt, Allemagne). Ainsi, selon un mode de réalisation avantageux, les coeurs flottants présentent une résistance mécanique, telle que mesurée par leur friabilité selon un "test de friabilité" défini ci-après dans le chapitre TESTS, qui est inférieure à 30 %, de préférence inférieure à 20 %, et plus préférentiellement inférieure à 10 %.

Certains excipients pharmaceutiques peuvent présenter, de par leur procédé de fabrication, une masse volumique apparente inférieure ou égale à 0,6 g/mL, une flottabilité dans des milieux aqueux d'au moins 50% pendant au moins 1 heure, une friabilité inférieure à 30 %, un diamètre moyen en volume compris entre 50 μm et 4000 μm, une porosité fermée supérieure ou égale à 0,2 et peuvent être aisément tamisés.

Tel est le cas notamment de l'acétate phtalate de cellulose (cellacefate) commercialisé par la société Eastman Chemical Company sous la marque Eastman™ C-A-P Cellulose Ester NF® (Drug Master File #8 de la FDA) sous la forme de granules ou de poudre. Il est connu d'utiliser l'acétate phtalate de cellulose commercialisé par la Société Eastman Chemical comme polymère d'enrobage dans des compositions pharmaceutiques.

Il est du mérite des inventeurs d'avoir trouvé qu'il était possible d'utiliser ce polymère commercialisé sous forme de granules ou de poudre, non pas comme polymère filmogène entérique, mais en tant que coeur flottant dans un système flottant de l'invention.

Ainsi, selon l'invention, dans la composition pharmaceutique de l'invention, les coeurs flottants sont composés d'acétate phtalate de cellulose, c'est-à-dire qu'ils comprennent au moins 50 % en poids d'acétate phtalate de cellulose, de préférence au moins 60 % en poids, de préférence au moins 70 % en poids, de préférence au moins 80 % en poids, de préférence au moins 90 % en poids, de préférence au moins 95% en poids, de préférence au moins 98 % en poids d'acétate phtalate de cellulose. Dans un mode de réalisation particulièrement avantageux, le coeur flottant selon l'invention est constitué d'acétate phtalate de cellulose, c'est-à-dire qu'il contient environ 100 % en poids d'acétate phtalate de cellulose. Par exemple, les coeurs flottants selon l'invention peuvent être des granules d'acétate phtalate de cellulose. Ces granules d'acétate phtalate de cellulose sont insolubles dans les milieux dont le pH est inférieur à 5,5 donc ils sont insolubles dans le milieu gastrique et sont des systèmes flottants au sens de la présente invention. Ils flottent par eux-mêmes, ce qui permet de déposer sur leur surface, par voie liquide, un principe actif et d'obtenir subséquemment des systèmes flottants sans qu'il soit nécessaire de déposer un film intermédiaire imperméabilisant.

Selon un mode de réalisation préféré, lesdits coeurs flottants sont des granules d'acétate phtalate de cellulose commercialisés par la société Eastman Chemical Company sous la marque Eastman™ C-A-P Cellulose Ester NF (Drug Master File # 8 de la FDA) sous forme de poudre ou bien sous forme de granules.

Avantageusement, les microparticules selon l'invention ne comprennent pas d'excipients effervescents.

**Principe actif :**

De telles compositions pharmaceutiques sont particulièrement avantageuses pour les principes actifs présentant une fenêtre d'absorption courte ou présentant une absorption préférentielle dans l'estomac. Elles peuvent également présenter un avantage pour les principes actifs ayant une activité locale dans l'estomac, comme pour le traitement local de l'hyperacidité gastrique et des ulcères de l'estomac ou du duodénum, et plus particulièrement pour le traitement contre la bactérie *Helicobacter pylori*. L'augmentation du temps de résidence dans l'estomac apportée par la présente invention peut permettre une meilleure efficacité de ces molécules actives et permettre ainsi de réduire la dose minimale nécessaire pour éradiquer ces bactéries.

Le principe actif est disposé sur la surface du coeur flottant, éventuellement à l'aide d'un liant, formant ainsi une couche de principe actif qui sera recouverte de l'enrobage à libération contrôlée. Ce liant permet une meilleure adhésion du principe actif sur le coeur flottant. Parmi les différents liants connus de l'homme de l'art, les polymères hydrosolubles sont utilisés selon un mode de réalisation avantageux de cette invention, tels que les polymères d'hydroxypropylcellulose d'hydroxypropylméthylcellulose, de povidone, les polysaccharides, les gommes d'acacia, d'Agar, de Caroube et semblables. La nature du liant sélectionné pourra dépendre de sa compatibilité chimique avec le principe actif. D'un point de vue quantitatif ce liant est utilisé dans des proportions de 5 à 95%, de préférence de 5 à 50% et plus préférentiellement encore de 5 à 20% de la couche de principe actif. La couche de principe actif peut également contenir d'autres excipients classiquement utilisés par l'homme du métier. On peut citer notamment les agents dispersants, les tensio-actifs, les conservateurs, les tampons, les agents protecteurs, les colorants, et leurs mélanges.

**Enrobage à libération contrôlée**

L'enrobage à libération contrôlée est appliqué sur les coeurs flottants recouverts de la couche de principe actif par des techniques connues de l'homme du métier, par exemple par la technique de l'enrobage par pulvérisation (spray coating) en lit d'air fluidisé.

Tout type d'enrobage conférant une libération contrôlée peut être mis en oeuvre dans les compositions pharmaceutiques de l'invention. En particulier, on peut citer les enrobages décrits notamment dans les demandes de brevet EP 0 709 087, WO 03/030878, EP 1 524 968, EP 1 524 969 et PCT/FR2009/050719 qui sont inclues par référence. L'homme du métier est capable d'ajuster la nature de l'enrobage et le taux de pelliculage pour obtenir le profil de dissolution souhaité.

Ainsi, selon une variante de l'invention, l'enrobage à libération contrôlée comprend :

- au moins un polymère P1 filmogène insoluble dans l'eau,
- au moins un polymère P2 soluble dans l'eau, et
- au moins un plastifiant PL,

Le polymère P1 filmogène insoluble dans l'eau peut être choisi dans le groupe constitué par les dérivés non hydrosolubles de la cellulose, notamment l'éthylcellulose, l'acétate de cellulose, l'acétate butyrate de cellulose ; les copolymères d'ammonio(méth)acrylate, notamment de type A (ex : Eudragit RL) ou de type B (ex : Eudragit RS) ; les copolymères d'éthylène et d'acétate de vinyle ; et leurs mélanges.

Le polymère P2 soluble dans l'eau peut être choisi dans le groupe constitué par la polyvinylpyrrolidone (PVP); les dérivés solubles de la cellulose tels que l'hydroxypropylméthyl cellulose (HPMC), la méthyl cellulose, l'hydroxyéthyl cellulose, l'hydroxyéthylméthyl cellulose, l'hydroxypropyl cellulose, la carboxyméthyl cellulose sodique ; l'isomalt ; la maltodextrine ; les poloxamers ; le polyéthylèneglycol ; le polyvinylalcool ; le copolymère vinylpyrrolidone-acétate de vinyle ; la gomme xanthane ; la gomme d'acacia ; la gomme carraghenane ; la gomme guar ; la gomme de caroube ; l'agar-agar ; le polydextrose ; les copolymères de méthylvinyl éther et d'anhydride maléique ou d'acide maléique; et leurs mélanges, de préférence ce polymère est la polyvinylpyrrolidone.

Le plastifiant PL est choisi dans le groupe constitué par les esters de glycérol, les phtalates, les citrates, les sébacates, notamment le sébacate de dibutyle, les esters d'alcool cétylique, l'huile de ricin, le polyéthylène glycol ; et leurs mélanges.

Plus spécifiquement, cet enrobage peut comprendre :

- 50 à 90 % de polymère P1 filmogène insoluble,
- 2 à 25 %, de préférence 5 à 15 %, de polymère P2 soluble dans l'eau, et
- 2 à 20 %, de préférence 4 à 15 % de plastifiant PL.

Plus particulièrement, dans cet enrobage, P1 est choisi dans le groupe constitué par l'éthylcellulose, l'acétate de cellulose, l'acétate butyrate de cellulose, les copolymères d'ammonio(meth)acrylate, les copolymères d'éthylène et d'acétate de vinyle, P2 est de préférence une polyvinylpyrrolidone et le plastifiant PL est de préférence l'huile de ricin ou le sébacate de dibutyle.

A titre d'exemple, un tel enrobage peut comprendre 50 à 90 % d'éthylcellulose, 2 à 25 % de polyvinylpyrrolidone et 2 à 20 % d'huile de ricin.

De préférence, les quantités de P1, P2 et PL satisfont aux caractéristiques suivantes : la fraction massique en poids sec de P1 par rapport à la masse totale de l'enrobage est comprise entre 40 et 90%, la fraction massique en poids sec P2/P1+P2 est comprise entre 15 et 60% et la fraction massique en poids sec PL/P1+PL est comprise entre 1 et 30%.

Selon une autre variante de l'invention, l'enrobage à libération contrôlée comprend :

- un polymère A sélectionné dans le groupe constitué par les dérivés de la cellulose : acétate phtalate de cellulose, phtalate d'hydroxypropyl méthylcellulose, acétate succinate de cellulose, acétate trimellitate de cellulose, acétate succinate d'hydroxypropyl méthylcellulose; carboxyméthyléthylcellulose ; les copolymères d'acide (méth)acrylique (EUDRAGIT ® S ou L) ; l'acétate phtalate de polyvinyle ; et leurs mélanges, et
- un composé B sélectionné dans le groupe constitué par les huiles végétales hydrogénées, les triglycérides, et leurs mélanges. Les triglycérides étant des produits naturels modifiés, il est entendu que les triglycérides peuvent contenir minoritairement notamment des mono- et/ou diglycérides,

- le rapport pondéral B/A étant compris entre 0,25 et 1,5, de préférence entre 0,5 et 1.

Cet enrobage permet une libération du principe actif qui est à la fois retardée et contrôlée.

Selon encore une autre variante, l'enrobage à libération contrôlée comprend un matériau composé d'au moins :

- 10 à 75 % en poids par rapport au poids total dudit enrobage d'au moins un polymère insoluble dans l'eau,
- 25 à 90 % en poids par rapport au poids total dudit enrobage d'au moins un polymère porteur de groupements carboxyliques libres et
- 0 à 25 % en poids par rapport au poids total dudit enrobage d'au moins un plastifiant,

lesdits polymères étant présents dans un rapport pondéral : [polymère(s) porteur de groupements carboxyliques libres / polymère(s) insoluble dans l'eau] au moins égal à 0,25.

Le polymère insoluble dans l'eau peut être choisi parmi l'éthylcellulose, l'acétate butyrate de cellulose, l'acétate de cellulose, les copolymères d'ammonio (méth)acrylate, notamment de type A ou de type B, les esters d'acides poly(méth)acryliques, et leurs mélanges et le polymère porteur de groupements carboxyliques libres peut être choisi parmi le(s) copolymère(s) d'acide méthacrylique et de méthacrylate de méthyle, le(s) copolymère(s) d'acide méthacrylique et d'acrylate d'éthyle, les dérivés cellulosiques tels que l'acétate phtalate de cellulose, l'acétate succinate de cellulose, l'acétate trimellilate de cellulose, le phtalate d'hydroxypropylméthylcellulose, l'acétate succinate d'hydroxypropylméthylcellulose, la carboxyméthyléthylcellulose ; la gomme shellac, l'acétate phtalate de polyvinyle ; et leurs mélanges.

De façon préférée, cet enrobage est formé d'au moins un mélange d'éthylcellulose, d'acétate butyrate de cellulose ou de copolymère d'ammonio (méth)acrylate de type « A » ou « B » avec au moins un copolymère d'acide méthacrylique et d'acrylate d'éthyle ou d'un copolymère d'acide méthacrylique et de méthacrylate de méthyle ou un de leurs mélanges.

L'enrobage à libération contrôlée, quel que soit le type choisi, peut en outre comprendre des additifs couramment utilisés par l'homme de l'art comme par exemple un lubrifiant ou un agent tensioactif, tels que le stéarate de magnésium ou l'huile de ricin hydrogénée polyoxyéthylénée.

Selon un mode de réalisation particulier de l'invention, il peut être nécessaire d'appliquer entre la couche de principe actif et la couche d'enrobage à libération contrôlée, une couche supplémentaire ayant un rôle de protection du principe actif, notamment vis-à-vis de l'oxygène ou de l'humidité de l'air, et/ou d'un des composants de la couche d'enrobage. Cette couche supplémentaire pourra être appliquée à raison de quelques pourcents massiques par des techniques connues de l'homme du métier, par exemple par la technique de l'enrobage par pulvérisation (spray coating) en lit d'air fluidisé et consistera principalement en un polymère filmogène choisi parmi les polymères connus de l'homme du métier pour leur rôle de protection, tels que les polymères d'hydroxypropylméthylcellulose ou de polyvinylalcool disponibles auprès de Colorcon Limited (Dartford UK).

**Taux de pelliculage :**

Dans la composition selon l'invention, les microparticules enrobées sont telles que ladite pellicule d'enrobage est suffisamment épaisse pour assurer une perméabilité contrôlée et une reproductibilité industrielle, et suffisamment mince pour ne pas modifier significativement la masse volumique apparente et permettre aux microparticules de flotter plusieurs heures. Ainsi, pour avoir à la fois la masse volumique apparente souhaitée et le profil de libération du principe actif souhaité, l'homme du métier peut ajuster le taux de pelliculage de l'enrobage à libération contrôlée ; le "taux de pelliculage Tp" étant la fraction massique de la couche d'enrobage à libération contrôlée par rapport à la masse totale de la microparticule enrobée, et étant calculé de la façon suivante :

$$Tp = \frac{poids\ de\ l'enrobage\ à\ libération\ contrôlée}{poids\ total\ des\ microparticules\ enrobées} \times 100$$

Selon un mode de réalisation particulier de l'invention, le taux de pelliculage Tp de la couche d'enrobage permettant le contrôle de la libération du principe actif est compris entre 5 et 50 %, de préférence entre 10 et 40 %, de plus préférentiellement encore entre 15 et 30 %.

Un tel taux de pelliculage est suffisant pour conférer le profil de libération du principe actif souhaité de manière industriellement reproductible, sans toutefois augmenter la masse volumique apparente des microparticules enrobées au-delà de 0,7 g/mL, de préférence au-delà de 0,6 g/mL, et plus préférentiellement encore au-delà de 0,5 g/mL, de façon à assurer une bonne flottabilité.

Ainsi, selon le choix de l'enrobage et de sa composition, il sera possible d'ajuster la libération du principe actif tout en conservant sa capacité à flotter et donc de synchroniser au mieux cette libération avec le temps de séjour dans l'estomac et la cinétique d'absorption du principe actif.

Compte tenu de la structure des microparticules enrobées mises en oeuvre dans la composition pharmaceutique de l'invention, il est possible d'une part de réguler la flottaison du coeur flottant en sélectionnant le coeur flottant par sa masse volumique apparente et/ou sa flottabilité et/ou sa porosité fermée, et d'autre part de moduler le contrôle de la libération du principe actif par la nature de l'enrobage à libération contrôlée et le taux de pelliculage, afin de pouvoir aisément synchroniser la durée de séjour dans l'estomac et la durée de libération pour optimiser l'efficacité du principe actif.

La composition pharmaceutique selon la présente invention se présente sous la forme de sachets, suspensions, gélules, comprimés ou comprimés orodispersibles pour administration orale. Outre les microparticules décrites précédemment, elle peut comprendre des excipients connus de l'homme du métier, tels que notamment des agents de compression, des liants, désintégrants, lubrifiants, viscosifiants, fluidifiants, colorants, édulcorants, etc.

La présente invention a également pour objet une microparticule enrobée à libération contrôlée présentant une masse volumique apparente inférieure ou égale à 0,7g/mL, comprenant un coeur à la surface duquel est déposée une couche contenant au moins un principe actif, ladite couche étant recouverte d'un enrobage à libération contrôlée, caractérisée en ce que ledit coeur est composé d'acétate phtalate de cellulose et présente une masse volumique apparente inférieure ou égale à 0,6g/mL.

La présente invention a également pour objet une microparticule enrobée à libération contrôlée comprenant un coeur flottant à la surface duquel est déposée une couche contenant au moins un principe actif, ladite couche étant recouverte d'un enrobage à libération contrôlée, caractérisée en ce que ledit coeur flottant est composé d'acétate phtalate de cellulose.

Les microparticules selon l'invention présentent avantageusement une flottabilité F telle que mesurée selon le test de flottaison décrit ci-après dans le chapitre TESTS, supérieure ou égale à 50 % après 1 heure, de préférence supérieure ou égale à 50 % après 4 heures, plus préférentiellement supérieure ou égale à 50 % après 6 heures et encore plus préférentiellement supérieure ou égale à 50 % après 8 heures.

La présente invention a également pour objet l'utilisation d'un coeur composé d'acétate phtalate de cellulose pour conférer à une microparticule comprenant ledit coeur une flottabilité F supérieure ou égale à 50 % après au moins 1 heure selon le test de flottaison tel que défini ci-après dans le chapitre TESTS.

La présente invention concerne également une méthode de traitement thérapeutique consistant essentiellement à administrer la composition de la présente invention par voie orale.

## Préparation des compositions pharmaceutiques

Le procédé de préparation des compositions de l'invention comprend les étapes successives suivantes :

a) sélection d'un coeur flottant,
b) tamisage sélectif éventuel
c) application du principe actif, éventuellement en présence d'un liant, à la surface du coeur flottant,
d) éventuellement application d'un film de protection,
e) application d'un enrobage à libération contrôlée.

Le procédé peut éventuellement comprendre une étape supplémentaire de mélange avec des excipients, éventuellement suivie d'une étape de compression.

L'étape a) de sélection des coeurs flottants se fait selon les propriétés telles qu'elles sont mentionnées ci-dessus, à savoir leur masse volumique apparente, leur taille, leur résistance mécanique et/ou leur porosité fermée.

Les étapes c), d) et e) sont réalisées à l'aide de techniques classiquement utilisées pour l'enrobage de particules, et notamment par enrobage par pulvérisation (spray coating) en lit d'air fluidisé.

Ce procédé de préparation peut être mis en oeuvre facilement sur tout type d'installation d'enrobage de microparticules.

L'invention est décrite plus en détail encore à l'aide des exemples et figures ci-après qui sont non limitatifs et donnés uniquement à titre illustratif.

## BREVE DESCRIPTION DES FIGURES

- La figure 1 est une représentation schématique, en section transversale, d'une microparticule enrobée mise en oeuvre dans les compositions pharmaceutiques de l'invention où 1 est la microparticule enrobée, 2 est le coeur flottant, 3 est la couche comprenant le principe actif et 4 est l'enrobage à libération contrôlée.

- La figure 2 est une photographie au microscope binoculaire de granules d'acétate phtalate de cellulose "Cellacefate Pellets" caractérisés dans l'exemple 1 de l'invention (grossissement 0,6 x 1,6).
- La figure 3 est une photographie au microscope binoculaire de la fraction 500-1000 $\mu$m obtenue par tamisage de l'acétate phtalate de cellulose "Cellacefate Powder" de l'exemple 2 de l'invention (grossissement 0,6 x 1,6).
- La figure 4 est une photographie au microscope binoculaire de granules d'acétate phtalate de cellulose enrobés avec un taux de pelliculage de 20%, préparés selon l'exemple 4 de l'invention.
- La figure 5 est un graphique représentant le pourcentage de carvédilol phosphate dissous dans HCl 0,1 N en fonction du temps pour les particules flottantes de l'exemple 4.
- La figure 6 est une photographie au microscope binoculaire de granules d'acétate phtalate de cellulose enrobés avec un taux de pelliculage de 20%, préparés selon l'exemple 6 de l'invention.
- La figure 7 est un graphique représentant le pourcentage de carvédilol phosphate dissous dans HCl 0,1 N en fonction du temps pour les particules flottantes préparées à l'exemple 6 à un taux de pelliculage de 20 % pour la courbe avec ronds noirs et à un taux de pelliculage de 15 % pour la courbe avec carrés noirs.
- La figure 8 est un graphique représentant le pourcentage d'ibuprofène dissous dans HCl 0,1 N en fonction du temps pour les particules flottantes préparées à l'exemple 8 (courbe avec croix).
- La figure 9 est une photographie au microscope binoculaire de granules d'acétate phtalate de cellulose enrobés avec un taux de pelliculage de 28%, préparés selon l'exemple 10 de l'invention.
- La figure 10 est un graphique représentant le pourcentage de metformine HCl dissous dans HCl 0,1 N + 0.5% tween 20 en fonction du temps pour les particules flottantes préparées à l'exemple 10 à un taux de pelliculage de 15 % pour la courbe avec triangles noirs et à un taux de pelliculage de 28 % pour la courbe avec carrés noirs.

**TESTS**

***Mesure de la masse volumique apparente après tassement ($\rho_a$)***

La méthode de mesure est basée sur l'essai du volume apparent décrit dans la pharmacopée européenne, édition 6.4, au chapitre 02.09.34. L'appareillage est constitué d'un appareil de tassement pouvant provoquer par minute 250 chutes d'une hauteur de 3mm et d'une éprouvette de 250 mL graduée. 100,0 g de poudre sont introduits dans l'éprouvette sèche. Si le volume correspondant est supérieur à 250 cm$^3$ alors une masse de 50 g seulement est introduite. Une lecture du volume de la poudre est effectuée après que 1250 chutes ont été subies. Le rapport entre la masse introduite et le volume lu correspond à la masse volumique apparente tassée, exprimée en g/mL.

***Mesure de la masse volumique particulaire ($\rho_p$)***

L'appareil utilisé est un pycnomètre à liquide. Le liquide utilisé est une solution d'acide chlorhydrique de normalité 0,1 N contenant 0,5 % (en poids) de tensioactif Tween 20 à la température ambiante. On pèse précisément le pycnomètre rempli du liquide ($M_1$). On pèse la prise d'échantillon de la poudre à mesurer ($M_2$). On pèse précisément le pycnomètre contenant la prise d'échantillon de la poudre à mesurer et complété avec le liquide ($M_3$). La masse volumique particulaire est calculée comme suit, $\rho_e$ étant la masse volumique du liquide.

$$\text{Masse volumique particulaire} = \frac{M_2 \rho_e}{M_1 + M_2 - M_3}$$

***Mesure de la flottaison F***

Les performances des compositions pharmaceutiques ou de leurs constituants (microparticules enrobées, coeurs flottants) sont mesurées à l'aide d'un test de flottaison qui permet d'évaluer non seulement leur aptitude à flotter à la surface du milieu aqueux dans lesquelles elles sont plongées, mais également leur aptitude à remonter à la surface après avoir été plongées au fond du récipient par agitation du milieu. Un tel test consiste à déverser 300 à 500 mg de produit à tester dans 500 mL d'une solution d'acide chlorhydrique de normalité 0,1 N contenant 0,5 % en poids de tensioactif Tween 80, maintenus à 37 °C pendant la durée du test. Un dispositif de dissolution tel qu'un dissolutest à palette décrit dans la pharmacopée européenne est utilisé. Le milieu est fortement agité à l'aide d'une spatule afin de permettre l'immersion totale du produit. Puis l'agitation est réglée à 50 tours/min pendant l'observation. Après 1 heure, 4 heures, 6 heures et/ou 8 heures, on retire les particules flottantes et on les pèse (on obtient une masse Mf) et on récupère les particules ayant coulé et on les pèse (on obtient une masse Mc), on calcule la valeur $F = \dfrac{Mf}{Mc + Mf} \times 100$

Les compositions pharmaceutiques de l'invention sont déclarées « systèmes flottants » si F est supérieure ou égale à 50 % après 1 heure, de préférence après 4 heures, plus préférentiellement encore après 6 heures et encore plus préférentiellement après 8 heures.

### Mesure du diamètre équivalent en volume D[4;3]

Le diamètre moyen est déterminé par diffraction laser ou analyse tamis selon l'échelle de taille à caractériser.
Jusqu'à une taille de 1000 $\mu$m, la distribution de tailles des particules est mesurée par diffraction laser à l'aide d'un appareil Mastersizer 2000 équipé d'un échantillonneur de poudre sèche. A partir de la répartition granulométrique mesurée sur une large gamme, le diamètre moyen équivalent en volume ou D(4;3) est calculé selon la formule suivante :

$$D(4;3) = \Sigma(d^4) / \Sigma(d^3)$$

Pour une taille supérieure à 1000 $\mu$m, la méthode par tamisage analytique est utilisée. Le choix de tamis sera aisément fait par l'homme du métier par référence à la Pharmacopée Européenne, édition 6.4, chapitre 02.09.38.

### Mesure de la friabilité

Le test de *friabilité* consiste à mesurer le diamètre D(4;3) à l'aide d'un granulomètre laser par voie sèche à une pression de 0,1 bar (soit $D_{0.1}$), puis à une pression de 2 bars (soit $D_2$), la friabilité étant égale à :

$$\frac{D_{0.1} - D_2}{D_{0.1}} \times 100$$

### EXEMPLES

Dans les exemples, les abréviations suivantes sont utilisées :

- CAP ou Cellacefate : acétate phtalate de cellulose, commercialisé par Eastman Chemicals Company (UK) sous la marque Eastman™ C-A-P Cellulose Ester NF
- Plasdone K29/32 : polyvinylpyrrolidone commercialisée par ISP
- Ethocel 7 premium : éthylcellulose commercialisée par Dow Chemicals
- TEC : citrate de triéthyle commercialisée par Morflex
- Eudragit L100-55 : copolymère d'acide méthacrylique/ méthacrylate de méthyle commercialisé par la société Evonik
- Lubritab : huile végétale hydrogénée/huile hydrogénée, commercialisé par la Société JRS Pharma

### Exemple 1 : caractérisation du CAP "Cellacefate Pellets"

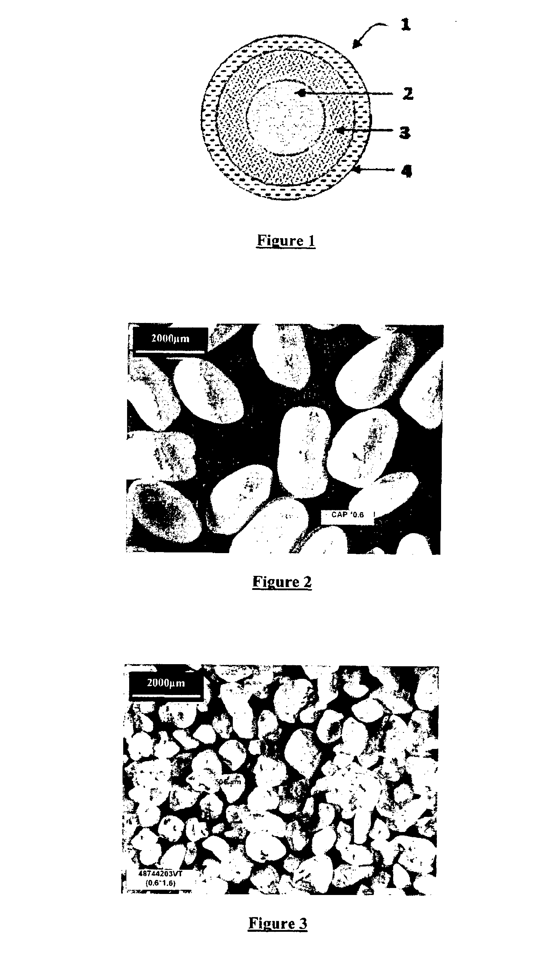

On observe les "Cellacefate Pellets" au microscope binoculaire et on les photographie (figure 2). Leur forme est ovoïde avec une surface lisse.
La masse volumique apparente $\rho_a$ du "Cellacefate Pellets" est déterminée selon la méthode décrite ci-dessus et est égale à 0,324 g/cm$^3$.
La masse volumique particulaire $\rho_p$ mesurée dans l'eau à l'aide d'un pycnomètre est égale à 0,514 g/cm$^3$.
La masse volumique vraie $\rho_v$ mesurée à l'aide d'un pycnomètre à hélium est égale à 1,3932 g/cm$^3$.
La porosité fermée est de 0,631 = 1-($\rho_p/\rho_v$)
La flottaison des "Cellacefate Pellets" est évaluée selon le test décrit ci-dessus. La totalité des particules restent à la surface du milieu aqueux après 24h.
La friabilité de ces pellets n'a pas pu être déterminée car la taille dépasse la limite supérieure de la plage d'utilisation de la machine (> 1000 $\mu$m). On estime qu'elle est analogue à celle de "Cellacefate Powder".

*Exemple 2: caractérisation du CAP "Cellacefate Powder"*

On effectue un tamisage de 100g de CAP "Cellacefate Powder" sur une colonne de tamis d'ouvertures de maille comprises entre 1000 et 50 µm. On mesure les masses retenues sur chaque tamis et on calcule les fractions de refus cumulés (tableau). Le D50 par tamisage est égal à environ 550µm.

| Ouverture de maille du tamis (µm) | % refus cumulés |
|---|---|
| 1000 | 11 |
| 800 | 23 |
| 630 | 42 |
| 500 | 55 |
| 400 | 63 |
| 300 | 76 |
| 200 | 87 |
| 50 | 100 |
| 0 | 100 |

On tamise manuellement 955 g de "Cellacefate Powder" sur des tamis équipés de toile de 50 µm, 500 µm et 1000 µm. On isole 499 g compris entre 500 et 1000 µm.

On observe cette fraction 500-1000 µm de "Cellacefate Powder " au microscope binoculaire (grossissement 0,6 x 1,6) et on les photographie (figure 3). Leur forme est ovoïde avec une surface lisse.

Le diamètre moyen en volume à 0,1 bar et 2 bars et la friabilité du "Cellacefate Powder" 500-1000 µm sont obtenus par granulométrie laser au Malvern voie sèche module Sirocco 2000, conformément au test décrit plus haut.

| Pression (en bar) | Diamètre [4;3] (en µm) |
|---|---|
| 0,1 | 973 |
| 2 | 948 |
| Friabilité : | 3% |

La masse volumique apparente du "Cellacefate Powder" 500-1000 µm est déterminée selon la méthode décrite ci-dessus et est égale à 0,290 g/cm$^3$.

La flottaison du "Cellacefate Powder" 500-1000 µm a été évaluée selon le test de flottaison décrit ci-dessus : 50 % des particules restent à la surface du milieu aqueux après 1h.

*Exemple 3 : Fabrication de granules flottants de carvédilol phosphate*

On prépare des granules flottants de carvédilol phosphate de la façon suivante. Dans un bécher en inox on introduit 360 mL d'eau déminéralisée puis 540 mL d'éthanol. Dans ce mélange, on introduit 60 g de Plasdone K29/32, et on agite pendant 15 minutes à 600 rpm (révolution par minutes). Puis on ajoute 240 g de carvédilol phosphate et on homogénéise le mélange. Dans un réacteur à lit d'air fluidisé du type GLATT GPCG1-1 on introduit 300 g de CAP sous forme de pellets commercialisés par la Société Eastman Chemicals sous la marque "Cellacefate pellets" et on pulvérise la totalité de la solution précédemment obtenue.

On obtient 531 g de granules flottants de carvédilol phosphate.

*Exemple 4: Fabrication de particules flottantes à libération contrôlée de carvédilol phosphate*

Dans un bécher en inox on introduit 2,12 g d'huile de ricin et 5,29 g de TEC, puis 426,18 g d'éthanol et on agite pendant 15 minutes à 600 rpm. On ajoute ensuite 9,53 g de Plasdone K29/32, 36 g d'Ethocel 7 premium sous agitation forte jusqu'à dissolution. On ajoute ensuite 182,65 g d'eau déminéralisée.

Dans un réacteur à lit d'air fluidisé du type Glatt GPCG1-1, on introduit 300g de granules flottants de carvédilol phosphate obtenus à **l'exemple 3** et on pulvérise la totalité de la solution préparée de façon à obtenir un taux de pelliculage tel que défini dans la partie description de 15%. On obtient 328 g de particules flottantes à libération contrôlée.

Ces particules sont représentées schématiquement à la Figure 1 sur laquelle la particule 1 comprend un substrat 2 sur lequel est déposée une couche 3 de principe actif, qui est elle-même enrobée d'une couche 4 à libération contrôlée. Cette figure, bien que décrite en lien avec l'exemple 1, représente de façon générale les particules mises en oeuvre dans les compositions de l'invention.

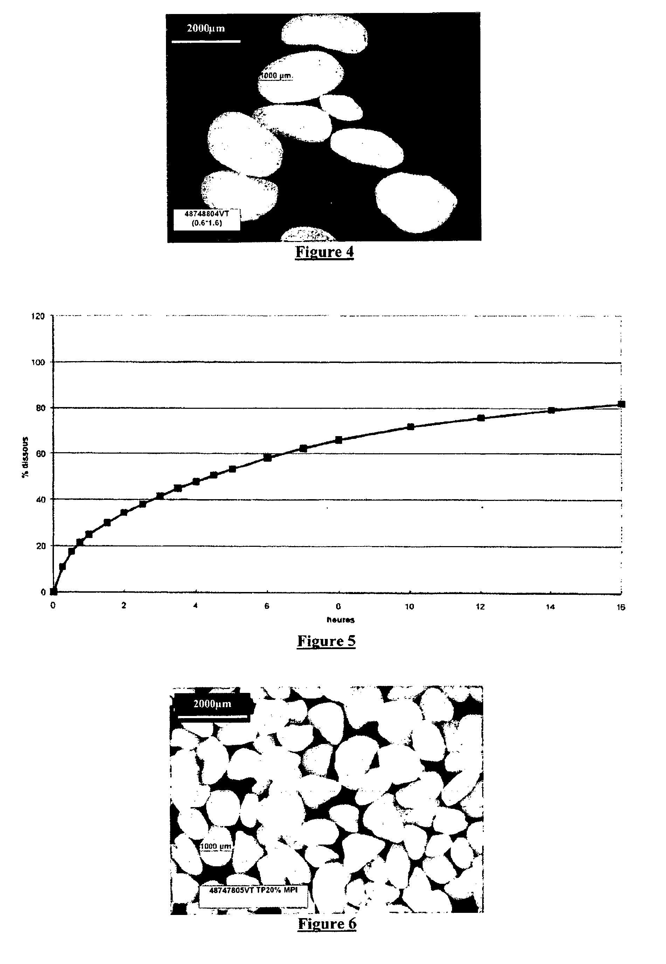

Ces particules sont photographiées sous microscope binoculaire (figure 4). Le pelliculage obtenu est uniforme sur toute la surface des particules.

La masse volumique apparente de ces particules est déterminée selon la méthode décrite ci-dessus et est égale à 0,429 g/cm$^3$.

Ces particules sont testées en flottaison selon le test décrit ci-dessus. Les résultats sont donnés dans le tableau ci-dessous. Au bout de 6h, encore 75% des particules flottent à la surface du milieu aqueux.

*Mesure de la dissolution du carvédilol phosphate*

| temps (heures) | Fraction de particules à la surface du milieu aqueux |
|---|---|
| 0 | 100% |
| 1 | 100% |
| 2 | 90% |
| 3 | 75% |
| 4 | 75% |
| 6 | 75% |

Le suivi de la dissolution du carvédilol phosphate dans le milieu HCl 0,1 N est effectué par mesure de l'absorbance UV à la longueur d'onde de 285 nm dans des cellules de 0,5 cm en comparaison avec une courbe d'étalonnage préalablement établie. Une quantité de particules correspondant à 80mg environ de carvédilol phosphate est introduite dans 900 mL de milieu, par bol d'appareil de dissolution, équipé de palettes d'agitation tournant à 100 rpm (appareil II de dissolution de la pharmacopée européenne) et maintenu à 37°C. Un prélèvement automatique est effectué à intervalles prédéterminés, et recyclé après lecture de l'absorbance. La courbe représentant la fraction de carvédilol dissoute en fonction du temps est présentée Figure 5.

### Exemple 5 : Fabrication de granules flottants de carvédilol phosphate

On procède comme dans l'exemple 3 mais en remplaçant les granules de CAP "Cellacefate pellets" par la fraction comprise entre 500 et 1000 μm obtenue par le tamisage de "Cellacefate powder".

On obtient 507 g de granules flottants de carvédilol phosphate à un taux d'enrobage de 50%.

### Exemple 6: Fabrication de microparticules flottantes de carvédilol phosphate à libération contrôlée

On procède comme dans l'exemple 4 mais en utilisant les granules flottants de carvédilol phosphate obtenus à l'exemple 5 et en pulvérisant une quantité de solution de pelliculage correspondant à un taux de pelliculage final de 20%.

Le pelliculage est uniforme sur toute la surface des microparticules. Les microparticules enrobées ainsi obtenues ont été observées au microscope binoculaire. La photographie correspondante est donnée en Figure 6.

La masse volumique apparente de ces particules, déterminée selon la méthode décrite ci-dessus, est égale à 0,457 g/cm$^3$.

Le diamètre moyen en volume est d'environ 1027 μm.

Ces particules sont testées en flottaison selon le test décrit ci-dessus. Les résultats sont donnés dans le tableau ci-dessous.

| temps (heures) | Fraction de particules à la surface du milieu aqueux |
|---|---|
| 0 | 100% |
| 1 | 90% |
| 2 | 75% |
| 3 | 75% |

(suite)

| temps (heures) | Fraction de particules à la surface du milieu aqueux |
|---|---|
| 4 | 67% |
| 5 | 50% |
| 6 | 25% |

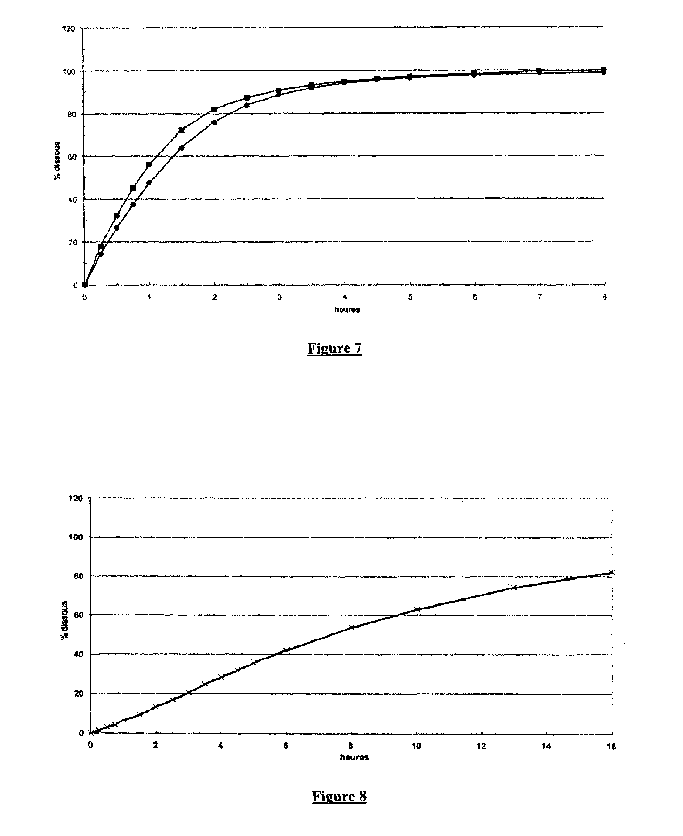

Le suivi de la dissolution du carvédilol phosphate dans le milieu HCl 0,1 N est effectué dans les même conditions que pour l'exemple 4. La courbe représentant la fraction de carvédilol dissoute en fonction du temps est présentée sur la courbe avec ronds noirs de la figure 7.

On procède de la même façon que ci-dessus, mais en pulvérisant une quantité de solution de pelliculage correspondant à un taux de pelliculage final de 15%. Le suivi de la dissolution du carvédilol phosphate est conduit comme ci-dessus et les résultats sont présentés sur la courbe avec carrés noirs de la figure 7.

### Exemple 7 : Fabrication de granules flottants d'ibuprofène

On prépare des granules flottants d'ibuprofène de la façon suivante. Dans un bécher en inox on introduit sous agitation 175 g d'acétone, 11,25 g de Klucel EF, puis on ajoute 63,75 g d'ibuprofène et on homogénéise le mélange. Dans un réacteur à lit d'air fluidisé du type GLATT GPCG1-1 on introduit 300 g de "Cellacefate pellets" et on pulvérise la totalité de la solution précédemment obtenue. On obtient 360 g de granules flottants d'ibuprofène.

Ces granules sont testés en flottaison selon le test décrit précédemment.

Après 20 heures, la totalité des granules flottent à la surface du milieu aqueux.

### Exemple 8: Fabrication de microparticules flottantes d'ibuprofène à libération contrôlée

Dans un bécher inox de taille adaptée, on introduit 6 g d'huile de ricin et 1,5 g de Crémophor RH40 dans 517,5 g d'acétone et 345 g d'isopropanol, sous agitation. Puis 7,5 g de Plasdone K29/32 sont ajoutés. Après dissolution, 60 g d'Ethocel 20, sont additionnés et on laisse sous forte agitation jusqu'à dissolution.

Dans un réacteur à lit d'air fluidisé de type GLATT GPCG1-1, on introduit des granules obtenus à l'exemple 7 ci-dessus, et on pulvérise la solution préparée précédemment, de façon à obtenir un taux de pelliculage de 20%. On obtient alors des microparticules d'ibuprofène à libération contrôlée.

La masse volumique apparente mesurée est égale à 0,363 g/cm$^3$.

Ces particules son testées en flottaison selon le test décrit ci-dessus : elles restent en totalité à la surface du milieu pendant 20 h au moins.

Le suivi de la dissolution de l'ibuprofène dans le milieu HCl 0,1 N est effectué dans les mêmes conditions que pour l'exemple 4. La courbe représentant la fraction d'ibuprofène dissoute en fonction du temps est présentée sur la figure 8.

### Exemple 9 : Fabrication de granules flottants de metformine HCl

On prépare des granules flottants de metformine HCl de la façon suivante. Dans un bécher en inox on laisse dissoudre sous agitation 500.0 g de metformine HCl dans 690.5 g d'eau. Dans un réacteur à lit d'air fluidisé du type GLATT GPCG1-1 on introduit 500 g de "Cellacefate pellets" et on pulvérise la totalité de la solution précédemment obtenue. On obtient 960 g de granules metformine HCl.

### Exemple 10: Fabrication de microparticules flottantes de metformine HCl à libération contrôlée

Dans un bécher inox de taille adaptée, on dissout sous agitation 30.0 g d'Eudragit L100-55 ; 15.0 g d'Eudragit S100 et 30.0 g de Lubritab dans 675.0 g d'isopropanol.

Dans un réacteur à lit d'air fluidisé de type GLATT GPCG1-1, on introduit 425.0 g des granules obtenus à l'exemple 9 ci-dessus, et on pulvérise la solution préparée précédemment. On obtient alors des microparticules de metformine HCl à libération contrôlée avec un taux de pelliculage de 15%.

Dans un second temps on pulvérise la même solution sur une fraction des microparticules obtenues afin d'obtenir un taux de pelliculage final de 28%.

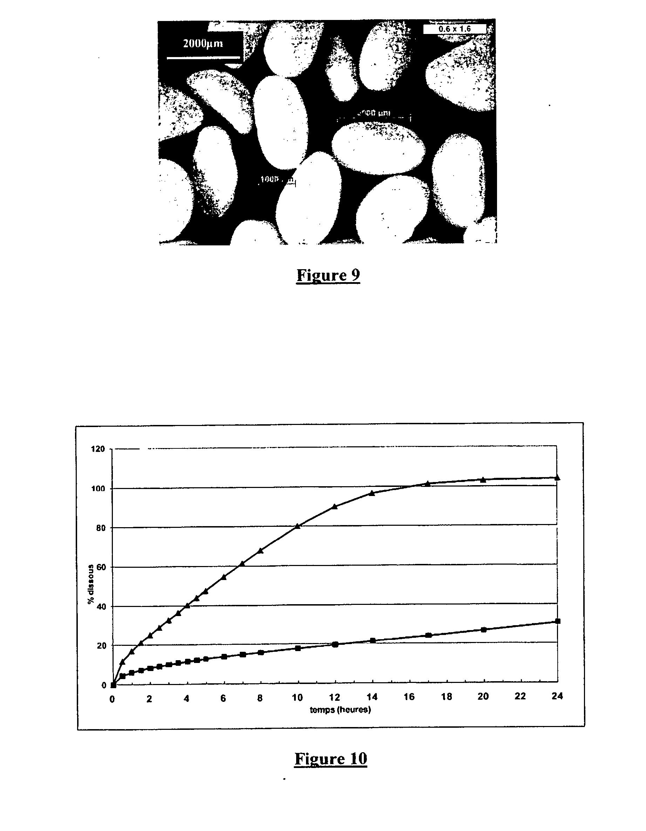

Les microparticules enrobées ainsi obtenues ont été observées au microscope binoculaire. La photographie correspondante est donnée en Figure 9.

La masse volumique apparente mesurée est égale à 0,52 g/cm$^3$.

Ces deux lots de particules sont testés en flottaison selon le test décrit ci-dessus : 95% des particules restent à la surface du milieu pendant 8 h et 70% sont encore présentes en surface après 24h.

Le suivi de la dissolution de la metformine HCl dans un milieu HCl 0,1 N + 0.5% Tween 20 est effectué par mesure de l'absorbance UV à la longueur d'onde de 232 nm dans des cellules de 0,1 cm en comparaison avec une courbe d'étalonnage préalablement établie. Une quantité de particules est introduite dans 900 mL de milieu, par bol d'appareil de dissolution, équipé de palettes d'agitation tournant à 100 rpm (appareil II de dissolution de la pharmacopée européenne) et maintenu à 37°C. Un prélèvement automatique est effectué à intervalles prédéterminés, et recyclé après lecture de l'absorbance.

Les courbes représentant la fraction cumulée de metformine HCl dissoute en fonction du temps sont présentées à la Figure 10 (triangles noirs pour le taux de pelliculage de 15% ; carrés noirs pour le taux de pelliculage de 28 %).

## Revendications

1. Composition pharmaceutique comprenant une pluralité de microparticules enrobées à libération contrôlée comportant chacune un coeur flottant, à la surface duquel est déposée une couche contenant au moins un principe actif, ladite couche étant recouverte d'un enrobage à libération contrôlée, **caractérisée par le fait que** ledit coeur flottant comprend au moins 50% en poids d'acétate phtalate de cellulose et présente une masse volumique apparente après tassement comme décrit dans la pharmacopée européenne, édition 6.4, au chapitre 02.09.34, inférieure ou égale à 0,6 g/mL et lesdites microparticules enrobées présentent une masse volumique apparente après tassement comme décrit dans la pharmacopée européenne, édition 6.4, au chapitre 02.09.34, inférieure ou égale à 0,7 g/mL.

2. Composition pharmaceutique selon la revendication 1, **caractérisée par le fait que** les coeurs flottants sont poreux et présentent une flottabilité F, telle que mesurée selon le test de flottaison, supérieure ou égale à 50 % après 1 heure.

3. Composition pharmaceutique selon la revendication 1 ou la revendication 2, **caractérisée par le fait que** le coeur flottant présente une porosité fermée supérieure ou égale à 0,2.

4. Composition pharmaceutique selon l'une quelconque des revendications 1 à 3, **caractérisée par le fait que** lesdits coeurs flottants présentent une résistance mécanique, telle que mesurée par le test de friabilité défini dans la description, inférieure à 30 %.

5. Composition pharmaceutique selon l'une quelconque des revendications 1 à 4, **caractérisée par le fait que** le diamètre moyen équivalent en volume desdits coeurs flottants est compris dans l'une des plages suivantes 50 à 500 μm, 500 à 1000 μm ou 1000 à 4000 μm.

6. Composition pharmaceutique selon l'une quelconque des revendications 1 à 5, **caractérisée par le fait que** lesdits coeurs flottants sont des granules d'acétate phtalate de cellulose.

7. Composition pharmaceutique selon l'une quelconque des revendications 1 à 6, **caractérisée par le fait que** lesdits coeurs flottants sont des granules d'acétate phtalate de cellulose, ledit acétate phtalate de cellulose comprenant 30 à 36% de groupements phtalate, 21.5 à 26% d'acétatate, une teneur en eau maximum de 0.5% en poids et une teneur maximum de 0.3% en poids en groupements acide libre.

8. Composition pharmaceutique selon l'une quelconque des revendications 1 à 7, **caractérisée par le fait que** l'enrobage à libération contrôlée comprend :

   - 50 à 90 % de polymère P1 insoluble dans l'eau,
   - 2 à 25 % de polymère P2 hydrosoluble, et
   - 2 à 20 % de plastifiant PL.

9. Composition pharmaceutique selon la revendication 8 **caractérisée par le fait que** :

   - le polymère filmogène P1 insoluble dans l'eau est choisi dans le groupe constitué par les dérivés non hydrosolubles de la cellulose, notamment l'éthylcellulose, l'acétate de cellulose, l'acétate butyrate de cellulose ; les copolymères d'ammonio(méth)acrylate ; les copolymères d'éthylène et d'acétate de vinyle ; et leurs mélanges ;
   - le polymère P2 soluble dans l'eau est choisi dans le groupe constitué par la polyvinylpyrrolidone (PVP) ; les dérivés solubles de la cellulose tels que l'hydroxypropylméthyl cellulose (HPMC), la méthylcellulose, l'hydroxyéthyl cellulose, l'hydroxyéthylméthyl cellulose, l'hydroxypropyl cellulose, la carboxyméthyl cellulose sodique ; l'isomalt ; la maltodextrine ; les poloxamers ; le polyéthylèneglycol ; le polyvinylalcool ; le copolymère vinylpyrrolidone-acétate de vinyle ; la gomme xanthane ; la gomme d'acacia ; la gomme carraghenane ; la gomme guar ; la gomme de caroube ; l'agar-agar ; le polydextrose ; les copolymères de méthylvinyl éther et d'anhydride maléique ou d'acide maléique ; et leurs mélanges, de préférence ce polymère étant la polyvinylpyrrolidone ; et
- le plastifiant PL est choisi dans le groupe constitué par les esters de glycérol, les phtalates, les citrates, les sébacates, notamment le sébacate de dibutyle, les esters d'alcool cétylique, l'huile de ricin, le polyéthylène glycol ; et leurs mélanges.

**10.** Composition pharmaceutique selon les revendications 8 ou 9, **caractérisée par le fait que** les quantités de P1, P2 et PL satisfont aux caractéristiques suivantes : la fraction massique en poids sec de P1 par rapport à la masse totale de l'enrobage est comprise entre 40 et 90%, la fraction massique en poids sec P2/P1+P2 est comprise entre 15 et 60% et la fraction massique en poids sec PL/P1+PL est comprise entre 1 et 30%.

**11.** Composition pharmaceutique selon l'une quelconque des revendications 1 à 7, **caractérisée par le fait que** l'enrobage à libération contrôlée comprend :

- un polymère A sélectionné dans le groupe constitué par les dérivés de la cellulose : acétate phtalate de cellulose, phtalate d'hydroxypropyl méthylcellulose, acétate succinate de cellulose, acétate trimellitate de cellulose, acétate succinate d'hydroxypropyl méthylcellulose, la carboxyméthyléthylcellulose ; les copolymères d'acide (méth)acrylique ; l'acétate phtalate de polyvinyle ; et leurs mélanges, et
- un composé B sélectionné dans le groupe constitué par les huiles végétales hydrogénées, les triglycérides, et leurs mélanges,
- le rapport pondéral B/A étant compris entre 0,25 et 1,5, de préférence entre 0,5 et 1.

**12.** Composition pharmaceutique selon l'une quelconque des revendications 1 à 7, **caractérisée par le fait que** l'enrobage à libération contrôlée comprend un matériau composé d'au moins :

- 10 à 75 % en poids par rapport au poids total dudit enrobage d'au moins un polymère insoluble dans l'eau, qui, de préférence est choisi parmi l'éthylcellulose, l'acétate butyrate de cellulose, l'acétate de cellulose, les copolymères d'ammonio (méth)acrylate, les esters d'acides poly(méth)acryliques, et leurs mélanges ;
- 25 à 90 % en poids par rapport au poids total dudit enrobage d'au moins un polymère porteur de groupements carboxyliques libres qui de préférence est choisi parmi le(s) copolymère(s) d'acide méthacrylique et de méthacrylate de méthyle, le(s) copolymère(s) d'acide méthacrylique et d'acrylate d'éthyle, les dérivés cellulosiques tels que l'acétate phtalate cellulose, l'acétate succinate de cellulose, l'acétate trimellilate cellulose, le phtalate d'hydroxypropylméthyl cellulose, l'acétate succinate d'hydroxypropylméthyl cellulose la carboxyméthyléthylcellulose; la gomme shellac ; , l'acétate phtalate de polyvinyle ; et leurs mélanges ; et
- 0 à 25 % en poids par rapport au poids total dudit enrobage d'au moins un plastifiant, lesdits polymères étant présents dans un rapport pondéral [polymère(s) porteur de groupements carboxyliques libres / polymère(s) insoluble dans l'eau] au moins égal à 0,25.

**13.** Microparticule enrobée à libération contrôlée comprenant un coeur flottant à la surface duquel est déposée une couche contenant au moins un principe actif, ladite couche étant recouverte d'un enrobage à libération contrôlée, **caractérisée en ce que** ledit coeur flottant comprend au moins 50% en poids d'acétate phtalate de cellulose, et présente une masse volumique apparente inférieure ou égale à 0.6g/ml et ladite microparticule enrobée présente une masse volumique apparente inférieure ou égale à 0,7g/mL.

**14.** Microparticule enrobée à libération contrôlée selon la revendication 13, **caractérisée en ce qu'**elle présente une flottabilité F, telle que mesurée selon le test de flottaison défini dans la description, supérieure ou égale à 50 % après 1 heure.

**15.** Utilisation d'un coeur composé d'acétate phtalate de cellulose pour conférer à une microparticule comprenant ledit coeur une flottabilité F supérieure ou égale à 50 % après 1 heure selon le test de flottaison tel que défini dans la description.

**EP 2 435 030 B1**

**Patentansprüche**

1. Pharmazeutische Zusammensetzung, umfassend mehrere überzogene Mikropartikel mit kontrollierter Freisetzung, jeweils umfassend einen schwimmfähigen Kern, auf dessen Oberfläche eine mindestens einen Wirkstoff enthaltende Schicht abgeschieden ist, wobei die Schicht mit einem Überzug mit kontrollierter Freisetzung bedeckt ist, **dadurch gekennzeichnet, dass** der schwimmfähige Kern mindestens 50 Gew.-% Celluloseacetatphtalat umfasst und eine Stampfdichte gemäß der Europäischen Pharmakopöe, Ausgabe 6.4, Kapitel 02.09.34, kleiner gleich 0,6 g/mL aufweist und die überzogenen Mikropartikel eine Stampfdichte gemäß der Europäischen Pharmakopöe, Ausgabe 6.4, Kapitel 02.09.34, kleiner gleich 0,7 g/mL aufweisen.

2. Pharmazeutische Zusammensetzung nach Anspruch 1, **dadurch gekennzeichnet, dass** die schwimmfähigen Kerne porös sind und eine Schwimmfähigkeit F gemäß dem Schwimmfähigkeitstest größer gleich 50% nach 1 Stunde aufweisen.

3. Pharmazeutische Zusammensetzung nach Anspruch 1 oder Anspruch 2, **dadurch gekennzeichnet, dass** der schwimmfähige Kern eine geschlossene Porosität größer gleich 0,2 aufweist.

4. Pharmazeutische Zusammensetzung nach einem der Ansprüche 1 bis 3, **dadurch gekennzeichnet, dass** die schwimmfähigen Kerne eine mechanische Festigkeit gemäß dem in der Beschreibung definierten Friabilitätstest von weniger als 30% aufweisen.

5. Pharmazeutische Zusammensetzung nach einem der Ansprüche 1 bis 4, **dadurch gekennzeichnet, dass** der mittlere volumenäquivalente Durchmesser der schwimmfähigen Kerne in einem der folgenden Bereiche liegt: 50 bis 500 $\mu$m, 500 bis 1000 $\mu$m oder 1000 bis 4000 $\mu$m.

6. Pharmazeutische Zusammensetzung nach einem der Ansprüche 1 bis 5, **dadurch gekennzeichnet, dass** es sich bei den schwimmfähigen Kernen um Celluloseacetatphtalatgranulat handelt.

7. Pharmazeutische Zusammensetzung nach einem der Ansprüche 1 bis 6, **dadurch gekennzeichnet, dass** es sich bei den schwimmfähigen Kernen um Celluloseacetatphtalat-Körner handelt, wobei das Celluloseacetatphtalat 30 bis 36% Phthalatgruppen, 21,5 bis 26% Acetatgruppen, einen Wassergehalt von maximal 0,5 Gew.-% und einen Maximalgehalt an freien Säuregruppen von 0,3 Gew.-% umfasst.

8. Pharmazeutische Zusammensetzung nach einem der Ansprüche 1 bis 7, **dadurch gekennzeichnet, dass** der Überzug mit kontrollierter Freisetzung

    - 50 bis 90% wasserunlösliches Polymer P1,
    - 2 bis 25% wasserlösliches Polymer P2 und
    - 2 bis 20% Weichmacher PL

    umfasst.

9. Pharmazeutische Zusammensetzung nach Anspruch 8, **dadurch gekennzeichnet, dass**:

    - das wasserunlösliche filmbildende Polymer P1 aus der Gruppe bestehend aus nicht wasserlöslichen Cellulosederivaten, insbesondere Ethylcellulose, Celluloseacetat, Celluloseacetatbutyrat; Ammonium(meth)acrylat-Copolymeren; Copolymeren von Ethylen und Vinylacetat und Mischungen davon ausgewählt ist;
    - das wasserlösliche Copolymer P2 aus der Gruppe bestehend aus Polyvinylpyrrolidon (PVP); löslichen Cellulosederivaten wie Hydroxypropylmethylcellulose (HMPC), Methylcellulose, Hydroxyethylcellulose, Hydroxyethylmethylcellulose, Hydroxypropylcellulose, Natriumcarboxymethylcellulose; Isomalt; Maltodextrin; Poloxameren; Polyethylenglykol; Polyvinylalkohol; Vinylpyrrolidon-Vinylacetat-Copolymer; Xanthangummi; Gummi arabicum; Carrageengummi; Guargummi; Johannisbrotkernmehl; Agar-Agar; Polydextrose; Copolymeren von Methylvinylether und Maleinsäureanhydrid oder Maleinsäure und Mischungen davon ausgewählt ist, wobei es sich bei diesem Polymer vorzugsweise um Polyvinylpyrrolidon handelt; und
    - der Weichmacher PL aus der Gruppe bestehend aus Glycerinestern, Phthalaten, Citraten, Sebacaten, insbesondere Dibutylsebacat, Cetylalkoholestern, Rizinusöl, Polyethylenglykol und Mischungen davon ausgewählt ist.

**10.** Pharmazeutische Zusammensetzung nach Anspruch 8 oder 9, **dadurch gekennzeichnet, dass** die Mengen von P1, P2 und PL den folgenden Merkmalen entsprechen: der Trockengewichts-Massenanteil von P1, bezogen auf die Gesamtmasse des Überzugs, liegt zwischen 40 und 90%, der Trockengewichts-Massenanteil P2/P1+P2 liegt zwischen 15 und 60% und der Trockengewichts-Massenanteil PL/P1+PL liegt zwischen 1 und 30%.

**11.** Pharmazeutische Zusammensetzung nach einem der Ansprüche 1 bis 7, **dadurch gekennzeichnet, dass** der Überzug mit kontrollierter Freisetzung

- ein Polymer A aus der Gruppe bestehend aus den Cellulosederivaten: Celluloseacetatphtalat, Hydroxypropylmethylcellulosephthalat, Celluloseacetatsuccinat, Celluloseacetattrimellitat, Hydroxypropylmethylcelluloseacetatsuccinat, Carboxymethylcellulose; (Meth)acrylsäure-Copolymeren; Polyvinylacetatphthalat und Mischungen davon und
- eine Verbindung B aus der Gruppe bestehend aus hydrierten Pflanzenölen, Triglyceriden und Mischungen davon

umfasst,
- wobei das Gewichtsverhältnis B/A zwischen 0,25 und 1,5 und vorzugsweise zwischen 0,5 und 1 liegt.

**12.** Pharmazeutische Zusammensetzung nach einem der Ansprüche 1 bis 7, **dadurch gekennzeichnet, dass** der Überzug mit kontrollierter Freisetzung ein Material umfasst, das mindestens aus

- 10 bis 75 Gew.-%, bezogen auf das Gesamtgewicht des Überzugs, mindestens eines wasserunlöslichen Polymers, das vorzugsweise aus Ethylcellulose, Celluloseacetatbutyrat, Celluloseacetat, Ammonium(meth)acrylat-Copolymeren, Poly(meth)acrylsäureestern und Mischungen davon ausgewählt ist;
- 20 bis 90 Gew.-%, bezogen auf das Gesamtgewicht des Überzugs, mindestens eines Polymers mit freien Carboxylgruppen, das vorzugsweise aus einem oder mehreren Copolymeren von Methacrylsäure und Methylmethacrylat, einem oder mehreren Copolymeren von Methacrylsäure und Ethylacrylat, Cellulosederivaten wie Celluloseacetatphtalat, Celluloseacetatsuccinat, Celluloseacetattrimellitat, Hydroxypropylmethylcellulosephthalat, Hydroxypropylmethylcelluloseacetatsuccinat, Carboxymethylcellulose; Schellack-Gummi; Polyvinylacetatphthalat und Mischungen davon ausgewählt ist; und
- 0-25 Gew.-%, bezogen auf das Gesamtgewicht des Überzugs, mindestens eines Weichmachers

besteht, wobei die Polymere in einem Gewichtsverhältnis [Polymer bzw. Polymere mit freien Carboxylgruppen / wasserunlösliches Polymer bzw. wasserunlösliche Polymere] von mindestens 0,25 vorliegen.

**13.** Überzogenes Mikropartikel mit kontrollierter Freisetzung, umfassend einen schwimmfähigen Kern, auf dessen Oberfläche eine mindestens einen Wirkstoff enthaltende Schicht abgeschieden ist, wobei die Schicht mit einem Überzug mit kontrollierter Freisetzung bedeckt ist, **dadurch gekennzeichnet, dass** der schwimmfähige Kern mindestens 50 Gew.-% Celluloseacetatphtalat umfasst und eine Schüttdichte kleiner gleich 0,6 g/ml aufweist und das überzogene Mikropartikel eine Schüttdichte kleiner gleich 0,7 g/ml aufweist.

**14.** Überzogenes Mikropartikel mit kontrollierter Freisetzung nach Anspruch 13, **dadurch gekennzeichnet, dass** es eine Schwimmfähigkeit F gemäß dem in der Beschreibung definierten Schwimmfähigkeitstest größer gleich 50% nach 1 Stunde aufweist.

**15.** Verwendung eines Kerns aus Celluloseacetatphtalat zur Ausstattung eines den Kern umfassenden Mikropartikels mit einer Schwimmfähigkeit F größer gleich 50% nach 1 Stunde gemäß dem in der Beschreibung definierten Schwimmfähigkeitstest.

**Claims**

**1.** Pharmaceutical composition comprising a plurality of controlled-release coated microparticles each comprising a floating core, on the surface of which is deposited a layer containing at least one active principle, said layer being covered with a controlled-release coating, wherein said floating core comprises at least 50% by weight cellulose acetate phthalate and has an apparent density, as described in the European Pharmacopeia, edition 6.4, chapter 02.09.34, of less than or equal to 0.6 g/mL and said coated microparticles have an apparent density, as described in the European Pharmacopeia, edition 6.4, chapter 02.09.34, of less than or equal to 0.7 g/mL.

**2.** Pharmaceutical composition according to claim 1, wherein the floating cores are porous and have a floatability F, as measured according to the flotation test, greater than or equal to 50 % after 1 hour.

**3.** Pharmaceutical composition according to claim 1 or claim 2, wherein the floating core has a closed porosity greater than or equal to 0.2.

**4.** Pharmaceutical composition according to anyone of claims 1 to 3, wherein said floating cores have a mechanical strength, as measured by the friability test defined in the description, of less than 30 %.

**5.** Pharmaceutical composition according to anyone of claims 1 to 4, wherein the equivalent volume mean diameter of said floating cores is comprised within one of the following ranges 50 to 500 $\mu$m, 500 to 1000 $\mu$m or 1000 to 4000 $\mu$m.

**6.** Pharmaceutical composition according to anyone of claims 1 to 5, wherein said floating cores are cellulose acetate phthalate granules.

**7.** Pharmaceutical composition according to anyone of claims 1 to 6, wherein said floating cores are cellulose acetate phthalate granules, said cellulose acetate phthalate comprising 30 to 36% of phthate groups, 21.5 to 26% acetate groups, a maximum of 5.0 weight % of moisture and a maximum of 3.0 weight % of free acid groups.

**8.** Pharmaceutical composition according to anyone of claims 1 to 7, wherein the controlled-release coating comprises:

- 50 to 90 % water-insoluble polymer P1,
- 2 to 25 % water-soluble polymer P2, and
- 2 to 20 % plasticizer PL.

**9.** Pharmaceutical composition according to claim 8, wherein:

- the water-insoluble film-forming polymer P1 is chosen from the group consisting of water-insoluble cellulose derivatives, in particular ethylcellulose, cellulose acetate, cellulose acetate butyrate; ammonio (meth)acrylate copolymers; ethylene and vinyl acetate copolymers; and mixtures thereof,
- the water-soluble polymer P2 is chosen from group consisting of polyvinylpyrrolidone (PVP); soluble cellulose derivatives, such as hydroxypropylmethyl cellulose (HPMC), methylcellulose, hydroxyethyl cellulose, hydroxyethylmethyl cellulose, hydroxypropylcellulose, sodium carboxymethyl cellulose; isomalt; maltodextrin; poloxamers; polyethylene glycol; polyvinyl alcohol; vinylpyrrolidone-vinyl acetate copolymer; xanthan gum; acacia gum; carragheenan gum; guar gum; carob gum; agar-agar; polydextrose; methylvinyl ether and maleic anhydride or maleic acid copolymers; and mixtures thereof, said polymer preferably being polyvinylpyrrolidone; and
- the plasticizer PL is chosen from the group consisting of glyceryl esters, phthalates, citrates, sebacates, in particular dibutyl sebacate, cetyl alcohol esters, castor oil, polyethylene glycol; and mixtures thereof.

**10.** Pharmaceutical composition according to claim 8 or 9, wherein the amounts of P1, P2 and PL conform to the following characteristics: the weight fraction as dry weight of P1 relative to the total weight of the coating is comprised between 40 and 90 %, the weight fraction as dry weight P2/P1+P2 is comprised between 15 and 60 % and the weight fraction as dry weight PL/P1+PL is comprised between 1 and 30 %.

**11.** Pharmaceutical composition according to anyone of claims 1 to 7, wherein the controlled-release coating comprises:

- a polymer A chosen from the group consisting of cellulose derivatives: cellulose acetate phthalate, hydroxypropyl methylcellulose phthalate, cellulose acetate succinate, cellulose acetate trimellitate, hydroxypropyl methylcellulose acetate succinate; carboxymethyl ethylcellulose; (meth)acrylic acid copolymers; polyvinyl acetate phthalate; and mixtures thereof, and
- a compound B chosen from the group consisting of hydrogenated vegetable oils, triglycerides, and mixtures thereof,
- the weight ratio B/A being comprised between 0.5 and 1.

**12.** Pharmaceutical composition according to anyone of claims 1 to 7, wherein the controlled-release coating comprises a material composed of at least:

- 10 to 75 % by weight relative to the total weight of said coating of at least one water-insoluble polymer, which is preferably chosen from ethylcellulose, cellulose acetate butyrate, cellulose acetate, ammonio (meth)acrylate copolymers, poly(meth)acrylic acid esters, and mixtures thereof;

- 25 to 90 % by weight relative to the total weight of said coating of at least one polymer bearing free carboxyl groups, which is preferably chosen from methacrylic acid and methyl methacrylate copolymer(s), methacrylic acid and ethyl acrylate copolymer(s), cellulose derivatives such as cellulose acetate phthalate, cellulose acetate succinate, cellulose acetate trimellilate, hydroxypropyl methylcellulose phthalate, hydroxypropyl methylcellulose acetate succinate; carboxymethyl ethylcellulose; shellac gum; polyvinyl acetate phthalate; and mixtures thereof; and

- 0 to 25% by weight relative to the total weight of said coating of at least one plasticizer,

said polymers being present in a weight ratio of polymer(s) bearing free carboxyl groups weight relatively to water-insoluble polymer(s) weight at least equal to 0.25.

13. Controlled-release coated microparticle comprising a floating core on the surface of which is deposited a layer containing at least one active ingredient, said layer being covered with a controlled-release coating, wherein said floating core comprises at least 50% by weight cellulose acetate phthalate and has an apparent density of less than or equal to 0.6 g/mL and said coated microparticle has an apparent density of less than or equal to 0.7 g/mL.

14. Controlled-release coated microparticle according to claim 13, which has a floatability F, as measured according to the flotation test defined in the description, greater than or equal to 50 % after 1 hour.

15. Use of a core composed of cellulose acetate phthalate to provide a microparticle comprising said core with a floatability F greater than or equal to 50 % after 1 hour according to the flotation test as defined in the description.

**Figure 1**

**Figure 2**

**Figure 3**

**Figure 4**

**Figure 5**

**Figure 6**

**Figure 7**

**Figure 8**

**Figure 9**

**Figure 10**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0158424 A
- FR 2263744
- US 5626876 A
- WO 2006063858 A
- US 20080317841 A, Grenier
- US 4844905 A
- US 4101650 A
- WO 2007010400 A
- US 6261601 B
- WO 02105415 A
- EP 709087 A
- WO 03030878 A
- EP 1524968 A
- EP 1524969 A
- FR 2009050719 W
- EP 0709087 A

**Littérature non-brevet citée dans la description**

- **ROUGE N. et al.** *Pharm. Acta Helv,* 1998, vol. 73 (2), 81-87
- **JOSEPH NJ et al.** *Journal of Controlled Released,* 2002, vol. 79, 71-79
- **JAIN SUNIL K. et al.** *Drug Development and Industrial Pharmacy,* 2007, vol. 33, 381-391
- **STREUBEL A. et al.** *International Journal of Phannaceutics,* 2002, vol. 198, 279-292
- **CHOI BY et al.** *International Journal of Pharmaceutics,* 2002, vol. 239, 81-91
- **BULGARELLI E. et al.** *International Journal of Pharmaceutics,* 2000, vol. 198, 157-165
- pharmacopée européenne